# NEUE EUROPÄISCHE PATENTSCHRIFT

(11) **EP 2 347 770 B2**
(45) Veröffentlichungstag und Bekanntmachung des Hinweises auf die Entscheidung über den Einspruch: **23.10.2019**
(45) Hinweis auf die Patenterteilung: 08.03.2017
(21) Anmeldenummer: 10183870.4
(22) Anmeldetag: 07.06.2000
(51) Int. Cl.: A61K 47/54, A61K 49/00, A61K 51/00, A61P 35/00

(54) **Verfahren zur Herstellung einer injizierbaren Arzneimittelzubereitung**
Process for producing an injectable pharmaceutical preparation
Procédé de production d' une préparation pharmaceutique injectable

(30) Priorität: 09.06.1999 DE 19926154
(43) Veröffentlichungstag der Anmeldung: 27.07.2011
(62) Teilanmeldung aus: 06012171.2
(73) Patentinhaber: Vergell Medical S.A., 1204 Geneva (CH)
(72) Erfinder: Kratz, Felix, 79106, Freiburg (DE)
(74) Vertreter: Weickmann & Weickmann PartmbB

(56) Entgegenhaltungen:
- EP-A1- 1 054 039
- WO-A2-98/00171
- WO-A2-98/10794
- WO-A2-99/48536
- DE-A1- 19 636 889
- KRATZ F ET AL: "PREPARATION, CHARACTERIZATION AND IN VITRO EFFICACY OF ALBUMIN CONJUGATES OF DOXORUBICIN", BIOLOGICAL & PHARMACEUTICAL BULLETIN (OF JAPAN), PHARMACEUTICAL SOCIETY OF JAPAN, TOKYO, JP, Bd. 21, Nr. 1, Januar 1998 (1998-01), Seiten 56-61, XP000738275, ISSN: 0918-6158
- KRUEGER M ET AL: "Synthesis and stability of four maleimide derivatives of the anticancer drug doxorubicin for the preparation of chemoimmunoconjugates", CHEMICAL AND PHARMACEUTICAL BULLETIN, PHARMACEUTICAL SOCIETY OF JAPAN, TOKYO, JP, Bd. 45, Nr. 2, 1997, Seiten 399-401, XP002162561, ISSN: 0009-2363
- WILLNER D ET AL: "(6-MALEIMIDOCAPROYL)HYDRAZONE OF DOXORUBICIN - A NEW DERIVATIVE FORTHE PREPARATION OF IMMUNOCONJUGATES OF DOXORUBICIN", BIOCONJUGATE CHEMISTRY, ACS, WASHINGTON, DC, US, Bd. 4, 1993, Seiten 521-527, XP000783675, ISSN: 1043-1802
- BEYER U ET AL: "SYNTHESE VON NEUEN BIFUNKTIONELLEN MALEINIMIDVERBINDUNGEN ZUR HERSTELLUNG VON CHEMOIMMUNOKONJUGATEN SYNTHESIS OF NEW BIFUNCTIONAL MALCIMIDE COMPOUNDS FOR THE PREPARATION OF CHEMOIMMUNOCONJUGATES", MONATSHEFTE FUR CHEMIE, SPRINGER VERLAG. WIEN, AT, Bd. 128, 1997, Seiten 91-102, XP009023613, ISSN: 0026-9247
- WILLNER D. ET AL: "(6-MALEIMIDOCAPROYL)HYDRAZONE OF DOXORUBICIN - A NEW DERIVATIVE FORTHE PREPARATION OF IMMUNOCONJUGATES OF DOXORUBICIN", Bioconjugate Chemistry, vol. 4, 1 January 1993 (1993-01-01), pages 521-527, DOI: 10.1021/bc00024a015

## Beschreibung

Die Erfindung betrifft eine Substanz zur Verwendung als injizierbares Medikament zur Behandlung von Krebs, die aus einem Wirkstoff, einem Spacermolekül und wenigstens einem kovalent proteinbindenden Molekül besteht und nach dem Zusammenbringen mit dem Körper über das kovalent proteinbindende Molekül kovalent an Körperflüssigkeits- oder Gewebebestandteile bindet, so daß eine Transportform des Wirkstoffs vorliegt, der pH-abhängig, hydrolytisch oder enzymatisch im Körper unter Freisetzung des Wirkstoffs spaltbar ist.

Der Großteil der zur Zeit eingesetzten Pharmaka sind niedermolekulare Verbindungen und weisen nach systemischer Applikation eine hohe Plasmasowie Gesamtclearance auf. Des weiteren dringen sie aufgrund von Diffusionsvorgängen in die Gewebestrukturen des Körpers ein und weisen in der Regel eine gleichmäßige Bioverteilung auf. Beide Eigenschaften führen dazu, daß nur geringe Mengen des Pharmakons den Wirkort erreichen und das Pharmakon aufgrund seiner Verteilung auf das gesunde Gewebe des Körpers Nebenwirkungen hervorruft. Diese Nachteile sind besonders bei solchen Pharmaka ausgeprägt, die ein hohes zytotoxisches Potential besitzen, wie etwa Zytostatika, Immunsuppressiva oder Virostatika.

Um die Selektivität von niedermolekularen Pharmaka zu verbessern, werden mehrere Strategien verfolgt, beispielsweise die chemische Derivatisierung von Leitstrukturen, die Formulierung als Prodrugs oder die Kopplung der Pharmaka an Trägermoleküle. Die vorliegende Erfindung geht von solchen Konzepten aus, bei denen Pharmaka an körpereigene Makromoleküle chemisch gebunden wurden. Bekannt sind Konjugate, bei denen im allgemeinen Zytostatika an Serumproteine, vorwiegend an bestimmte Trägermoleküle wie Humanserumalbumin und Humanserumtransferrin, gebunden werden und dann verabreicht werden. Diese bekannten Proteinkonjugate werden dadurch hergestellt, daß man ex vivo entweder in einem "Eintopfverfahren" das Zytostatikum an das Serumprotein koppelt (DE 41 22 210 A1) und das resultierende Albumin-Zytostatikum-Konjugat gewinnt oder daß man zunächst das Zytostatikum mit einem geeigneten Spacermolekül derivatisiert, das resultierende Produkt isoliert und in einem zweiten Schritt das so derivatisierte Zytostatikum über eine Maleinimidgruppe an das Protein koppelt (DE 196 36 889 A1 und PCT/DE 97/02000) und das resultierende Albumin-Zytostatikum-Konjugat isoliert. Beide Verfahren haben den Nachteil, daß Plasmaproteine verwendet werden, die pathogene Erreger enthalten können. Weitere Nachteile der beschriebenen Protein-Wirkstoff-Konjugate sind ihre unbefriedigende Stabilität und Lagerbeständigkeit und der technische Aufwand ihrer Herstellung.

Der Erfindung liegt die Aufgabe zugrunde, diese Nachteile zu überwinden. Gelöst wird diese Aufgabe durch eine Substanz zur Verwendung als injizierbares Medikament zur Behandlung von Krebs, dadurch gekennzeichnet,dass die Substanz die Formel: 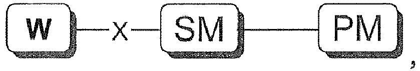 aufweist, worin
W ein Wirkstoff ist, wobei der Wirkstoff ein Zytostatikum ist,
SM ein Spacermolekül ist,
PM ein kovalent proteinbindendes Molekül ist,
X eine chemische Gruppe zwischen dem Wirkstoff und dem Spacer ist, ausgewählt aus: 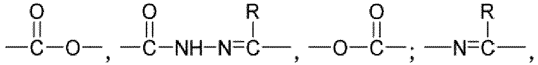 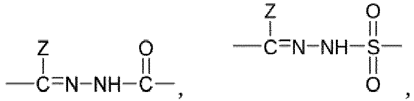 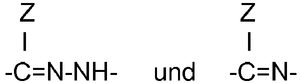 worin
   R = H, Alkyl, Phenyl oder substituiertes Phenyl, und
   Z = chemische Gruppe des Wirkstoffs ist.

Die Bindung zwischen dem Wirkstoff und dem Spacer ist pH-abhängig, hydrolytisch oder enzymatisch im Körper spaltbar. Bei der Spaltung wird der Wirkstoff oder ein Derivat des Wirkstoffes freigesetzt. Unter einem Wirkstoffderivat werden Substanzen verstanden, die den Wirkstoff umfassen, jedoch zusätzlich Teile des Spacers oder der Gruppen, über die der Wirkstoff mit dem kovalent proteinbindenden Molekül gebunden war, enthalten können. Die Wirksamkeit des Wirkstoffs sollte durch seine Freisetzung als
Derivat nicht beeinträchtigt werden. Vorzugsweise entfaltet der Wirkstoff bzw. dessen Derivat seine Wirksamkeit erst nach der Freisetzung.

Die Erfindung beruht auf der überraschenden Feststellung, daß es nicht, wie bisher angenommen wurde, nötig ist, einen Wirkstoff mit einem bestimmten Träger unter definierten Bedingungen zu verbinden und das Produkt zu verabreichen, sondern daß es möglich ist, therapeutisch und/oder diagnostisch wirksame Substanzen, bestehend aus einem pharmakologischen Wirkstoff, und wenigstens einem kovalent proteinbindenden Molekülteil, die durch einen Spacer miteinander verbunden sind, direkt als injizierbare Arzneimittel einzusetzen, da diese nach dem Zusammenbringen mit dem Körper über das kovalent proteinbindende Molekül kovalent derart an Körperflüssigkeits- oder Gewebebestandteile, vorwiegend an Serumproteine, binden, daß in vivo eine Transportform des Wirkstoffs gebildet wird, welche die Zielzellen bzw. das Zielgewebe des Wirkstoffs erreicht. Da erfindungsgemäß bei der therapeutisch wirksamen Substanz die Bindung zwischen dem Wirkstoff und dem Spacermolekül pH-abhängig, hydrolytisch oder enzymatisch im Körper spaltbar ist, wird der Wirkstoff trotzdem gezielt am gewünschten Zielort freigesetzt.

Substanzen zur erfindungsgemäßen Verwendung als injizierbares Medikament zur Behandlung von Krebs verändern und verbessern aufgrund ihrer kovalent proteinbindenden Eigenschaften das pharmakokinetische Profil der Wirkstoffe entscheidend. Wenn diese therapeutisch wirksamen Substanzen in Körperflüssigkeiten gelangen, binden sie kovalent an Körperflüssigkeits- oder Gewebebestandteile, vorzugsweise an Serumproteine, mehr bevorzugt an Serumalbumin, um so als makromolekulare Prodrugs vorzuliegen, die den Wirkstoff zu dem Zielort transportieren und/oder ihn in einer dosierten Form freisetzen.

Die Substanz zur erfindungsgemäßen Verwendung besteht aus einem Wirkstoff W, einem Spacermolekül SM und wenigstens einem kovalent proteinbindenden Molekül PM mit folgender allgemeiner Struktur:

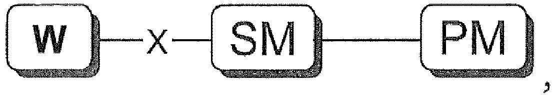

Zusätzlich kann die Substanz Markierungsgruppen bzw. markierte Elemente oder Moleküle aufweisen. Bevorzugte Marker sind ein oder mehrere Radionuklide, ein oder mehrere Radionuklide umfassende Liganden, eine oder mehrere Positronenstrahler, ein oder mehrere NMR-Kontrastmittel, ein oder mehrere fluoreszierende Verbindung(en) oder/und ein oder mehrere Kontrastmittel im nahen IR-Bereich.

Der Wirkstoff ist ein Zytostatikum.

Bevorzugte Zytostatika für die Substanz zur erfindungsgemäßen Verwendung sind die Anthrazykline Doxorubicin, Daunorubicin, Epirubicin, Idarubicin, Mitoxantron und Ametantron sowie verwandte Derivate, die Alkylantien Chlorambucil, Bendamustin, Melphalan und Oxazaphoshorine sowie verwandte Derivate, die Antimetabolite Methotrexat, 5-Fluorouracil, 5'-Desoxy-5-fluorouridin und Thioguanin sowie verwandte Derivate, die Taxane Paclitaxel und Docetaxel sowie verwandte Derivate, die Camptothecine Topotecan, Irinotecan, 9-Aminocamptothecin und Camptothecin sowie verwandte Derivate, die Podophyllotoxinderivate Etoposid, Teniposid und Mitopodozid sowie verwandte Derivate, die Vinca-Alkaloide Vinblastin, Vincristin, Vindesin und Vinorelbin sowie verwandte Derivate, die Calicheamicine, die Maytansinoide und eine Verbindung der allgemeinen Formel I bis XII:

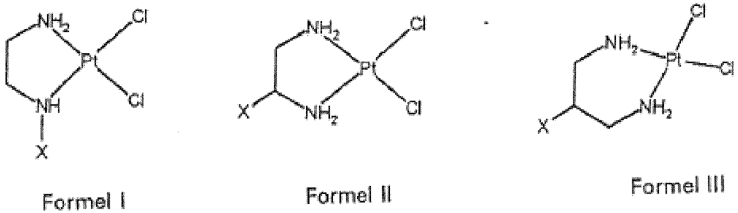

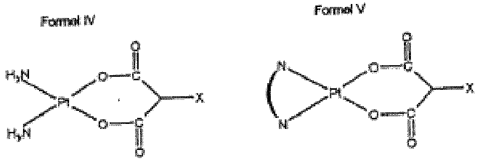

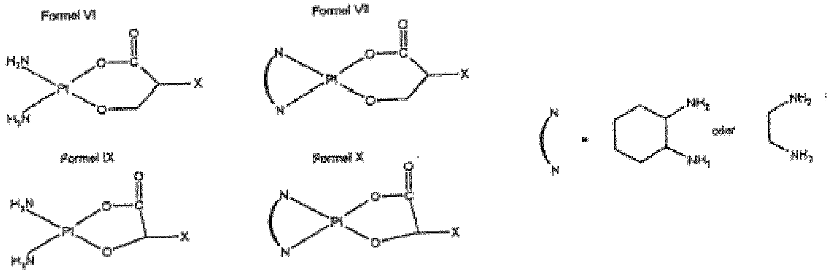

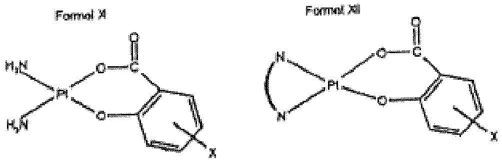

Das Spacermolekül SM ist ein organisches Molekül bestehend aus einer aliphatischen Kohlenstoffkette und/oder einem aliphatischen Kohlenstoffring und/oder mindestens einem Aromaten. Die/der aliphatische Kohlenstoffkette/-ring besteht vorzugsweise aus 1-12 Kohlenstoffatomen, die teilweise durch Sauerstoffatome ersetzt sein können, und kann gegebenenfalls substituiert sein, insbesondere durch eine oder mehrere wasserlösliche Gruppen, wie Sulfonsäure-, Aminoalkyl- oder Hydroxygruppen. Der Aromat ist vorzugsweise ein Benzolring, der gegebenenfalls substituiert sein kann, wie etwa mit den obengenannten wasserlöslichen Gruppen. Die aliphatische Kohlenstoffkette kann zur besseren Wasserlöslichkeit Sauerstoffatome enthalten und sich dazu zweckmäßig von einer Oligoethylenoxid- oder -propylenoxidkette ableiten, z.B. eine Diethylenglycol-, Triethylenglycol- oder Dipropylenglycolkette sein.

Das kovalent proteinbindende Molekül PM ist vorzugsweise eine Maleinimidgruppe, eine Halogenacetamidgruppe, eine Halogenacetatgruppe, eine Pyridyldithio-Gruppe, eine N-Hydroxysuccinimidester- oder eine Isothiocyanat-Gruppe. Es kann auch eine Disulfidgruppe, eine Vinylcarbonylgruppe, eine Aziridingruppe oder eine Acetylengruppe sein. Die Disulfidgruppe ist bevorzugt aktiviert, in der Regel dadurch, daß eine Thionitrobenzoesäure (z.B. 5'-Thio-2-Nitrobenzoesäure) die austauschbare Gruppe darstellt. Die Gruppen können gegebenenfalls substituiert sein. Die Maleinimid-, Pyridyldithio- bzw. N-Hydroxysuccinimidester-Gruppe kann gegebenenfalls mit Alkyl oder mit den obengenannten wasserlöslichen Gruppen substituiert sein. PM besitzt kovalent proteinbindende Eigenschaften, d.h. es bindet im physiologischen Milieu kovalent an bestimmte Aminosäuren auf der Proteinoberfläche. Dabei reagiert die Maleinimid-, die Halogenacetamid-, die Halogenacetat, die Pyridyldithio-Gruppe, die Disulfidgruppe, die Vinylcarbonylgruppe, die Aziridingruppe bzw. die Acetylengruppe vorzugsweise mit HS-Gruppen von Cysteinen, die N-Hydroxysuccinimidester- und Isothiocyanatgruppe reagiert vorzugsweise mit der Aminogruppe von Lysinen auf der Proteinoberfläche.

Die Substanz zur erfindungsgemäßen Verwendung gelangt nach parenteraler Applikation in die Blutbahn und kann über PM an Proteine binden. Bevorzugt erfolgt die Bindung an Serumproteine, insbesondere Serumalbumin. Es wurde gefunden, daß im physiologischen Milieu die therapeutisch und/oder diagnostisch wirksame Substanz über die Maleinimid-, Halogenacetamid-, Halogenacetat-, Pyridyldithio-Gruppe, die Disulfidgruppe, die Vinylcarbonylgruppe, die Aziridingruppe bzw. die Acetylengruppe insbesondere mit dem freien Cystein-34 des Albumins reagiert und auf diesem Weg kovalent gebunden wird. Pharmakologisch aktive Substanzen mit einer N-Hydroxysuccinimidester- bzw. Isothiocyanat-Gruppe binden bevorzugt an die ε-Aminogruppe von Lysinen auf der Proteinoberfläche von Albumin oder anderen Serumproteinen. Serumproteine, wie Albumin oder Transferrin, weisen eine ausgesprochen lange Halbwertszeit im systemischen Kreislauf auf (bis zu 19 Tagen - Peters, T. Jr. (1985): Serum albumin. Adv. Protein. Chem. 37, 161-245). Aufgrund einer erhöhten Permeabilität von Gefäßwänden des malignen, infizierten bzw. entzündeten Gewebes für Makromoleküle gelangt Serumalbumin bevorzugt in dieses Zielgewebe (Maeda, H.; Matsumura, Y. Crit. Rev. Ther. Drug Carrier Sys. (1989), 6, 193-210). Dadurch kann ein an Albumin gekoppelter Wirkstoff gezielter den Wirkort erreichen. Des weiteren verhindert die kovalente Kopplung des Wirkstoffs an Serumproteine in der Blutbahn, daß der Wirkstoff in gesunde Gewebestrukturen des Körpers diffundiert oder über die Niere eliminiert wird bzw. diese in dem Maße schädigt wie der nicht gebundene Wirkstoff. Dadurch wird das pharmakokinetische Profil des Wirkstoffs verändert und verbessert, da seine Wirkung durch eine Anreicherung am Wirkort erhöht wird und gleichzeitig die toxischen Wirkungen auf gesunde Systeme des Körpers verringert werden.

Die Substanzen zur Verwendung gemäß der vorliegenden Erfindung enthalten zwischen SM und W eine definierte chemische Gruppe X. Diese Gruppe ist pH-abhängig, vorzugsweise säurelabil, spaltbar bzw. hydrolytisch spaltbar, oder sie enthält mindestens eine Bindung, die im Körper enzymatisch gespalten wird.

Bindungen, die durch Hydrolyse unter Freisetzung des Wirkstoffs gespalten werden, sind beispielsweise Esterbindungen oder Metallkomplexbindungen, wie sie bei Platin-Dicarboxylat-Komplexen vorliegen, wobei ein Diammindiaquo-Platin(II)-Komplex freigesetzt wird. Die säurelabilen spaltbaren Bindungen sind Acetal-, Ketal-, Imin-, Hydrazon, Carboxylhydrazon- oder Sulfonylhydrazonbindungen oder cis-Aconitylbindungen oder eine Tritylgruppe enthaltende Bindungen, wobei die Tritylgruppe substituiert oder nicht substituiert sein kann. Bevorzugte therapeutisch/diagnostisch relevante säurelabile Bindungen sind beispielsweise in Kratz et al. (1990) Crit. Rev. Ther. Drug. Car.Sys. 16 (3), 245-288 beschrieben. Die Peptidsequenz in den realisierten Peptidbindungen besteht in der Regel aus etwa 2-30 Aminosäuren. Die Peptidsequenz ist dabei vorzugsweise auf die Substratspezifität bestimmter Enzyme, nachstehend als Targetenzyme bezeichnet, im Körper zugeschnitten, so daß die Peptidsequenz oder ein Teil diese Sequenz von einem Enzym im Körper erkannt wird und das Peptid gespalten wird. Gemäß einer weiteren Ausführungsform der vorliegenden Erfindung besteht die enzymatisch spaltbare Bindung aus einer Bindung, die keine Peptidbindung ist. Beispiele sind Carbamatbindungen, die durch krankheitsspezifische Enzyme, z.B. Glutathion-S-Transferasen, Glucuronidasen, Galactosidasen, den Wirkstoff oder ein Wirkstoffderivat freisetzen. Es ist auch ohne weiteres möglich, daß eine enzymatisch spaltbare Bindung aus einer Peptidsequenz und einer der obengenannten Bindungen, die keine Peptidbindung ist, aufgebaut ist. Die Targetenzyme können sowohl körpereigene Enzyme sein oder Enzyme, die in Mikroorganismen vorkommen bzw. von diesen gebildet werden.

Die Targetenzyme sind in der Regel Proteasen, Serinproteasen, Plasminogenaktivatoren und Peptidasen, beispielsweise Matrix-Metalloproteasen (MMP) oder Cysteinproteasen, die bei Erkrankungen wie rheumatoider Arthritis oder Krebs verstärkt gebildet oder aktiviert sind, was zum exzessiven Gewebeabbau, zu Entzündungen und zur Metastasierung führt. Targetenzyme sind insbesondere MMP 2, MMP3 und MMP 9, die als Proteasen bei den genannten pathologischen Prozessen beteiligt sind (Vassalli, J., Pepper, M.S. (1994), Nature 370, 14-15, Brown, P.D. (1995), Advan Enzyme Regul. 35, 291-301).

Weitere Proteasen, die Targetenzyme für therapeutisch und/oder diagnostisch wirksame Substanzen der vorliegenden Erfindung darstellen, sind Cathepsine, insbesondere Cathepsin B, H und L, die als
Schlüsselenzyme bei entzündlichen und malignen Erkrankungen identifiziert worden sind.

Die oben genannten Bindungstypen gewährleisten, daß der Wirkstoff oder ein entsprechend aktives Derivat am Wirkort extrazellulär und/oder intrazellulär nach Aufnahme des Konjugats durch die Zelle freigesetzt wird und seine therapeutische Wirkung entfalten kann.

Die Spaltung kann auch dergestalt ablaufen, dass nicht der Wirkstoff als solcher abgespalten wird, sondern ein Derivat des Wirkstoffs. Ein solches Derivat enthält somit den Wirkstoff sowie daran gebundene Gruppen, die aus dem Spacermolekül stammen, je nachdem, an welcher Stelle die gewünschte Spaltung erfolgt ist.

Die Substanz zur Verwendung gemäß der vorliegenden Erfindung kann gemäß einer der untenstehenden allgemeinen Beschreibungen hergestellt werden:
Wirkstoffe, die eine HOOC-Gruppe besitzen, werden folgendermaßen derivatisiert:

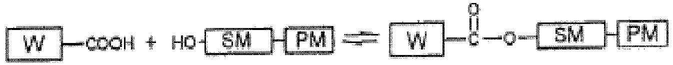

Die Veresterung erfolgt dabei durch übliche Verfahren, die dem Fachmann geläufig sind.

Es ist weiterhin möglich, die HOOC-Gruppe in eine Hydrazidgruppe zu überführen, z.B. durch Umsetzen mit tert.-Alkylcarbazaten und anschließende Spaltung mit Säuren (beschrieben in DE 196 36 889), und das eine Hydrazidgruppe aufweisende Pharmakon mit einem eine Carbonylkomponente enthaltenen Spacer, bestehend aus PM und SM, umzusetzen, wie u.a. in DE 196 36 889 A1 bzw. PCT/DE 97/02000 beschrieben ist:

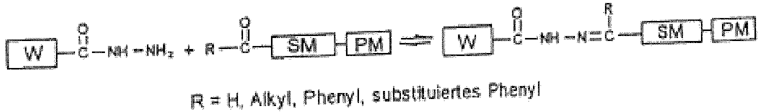

Wirkstoffe, die eine H₂N-Gruppe besitzen, werden folgendermaßen derivatisiert:

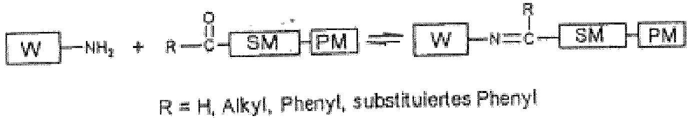

Die Reaktion zu den Iminderivaten erfolgt dabei durch übliche Verfahren, die dem Fachmann geläufig sind.

Wirkstoffe, die eine HO-Gruppe besitzen, werden folgendermaßen derivatisiert:

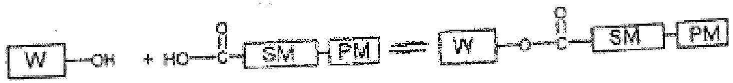

Die Veresterung erfolgt dabei durch übliche Verfahren, die dem Fachmann geläufig sind.

Wirkstoffe, die eine Carbonylkomponente besitzen, werden folgendermaßen derivatisiert:

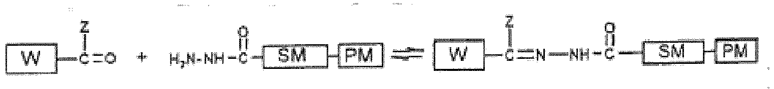

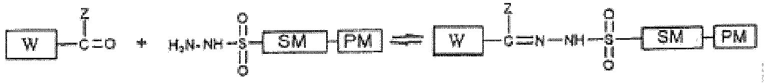

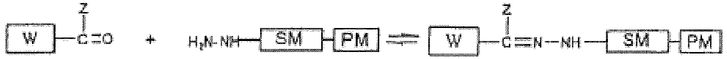

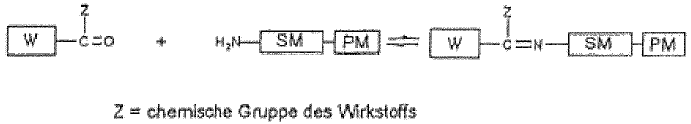

Die Umsetzung zu den Carboxyhydrazon-, Sulfonylhydrazon-, Hydrazon- bzw. Iminderivaten erfolgt dabei gemäß Verfahren, die u.a. in DE 196 36 889 A1 bzw. PCT/DE 97/02000 beschrieben sind, oder durch übliche Verfahren, die dem Fachmann geläufig sind.

Es ist weiterhin möglich, eine HO-Gruppe oder eine NH₂-Gruppe eines Wirkstoffs in eine Carbonylkomponente zu überführen, z.B. durch Veresterung bzw. Amidbildung mit einer Carbonsäure-tragenden Carbonylkomponente gemäß folgender allgemeiner Formel: 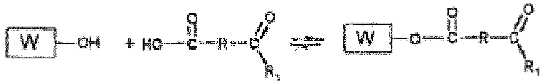 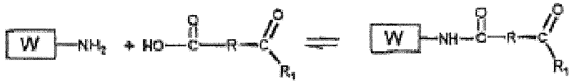 wobei R eine aliphatische Kohlenstoffkette und/oder ein aliphatischer Kohlenstoffring und/oder ein Aromat und R₁ = H, Alkyl-, Phenyl- oder eine substituierte Phenylgruppe ist. R besteht in der Regel aus 1 bis 12 Kohlenstoffatomen, die gegebenenfalls substituiert sein können, z.B. durch wasserlösliche Gruppen, wie Sulfonsäure-, Aminoalkyl- oder Hydroxygruppen. Der Aromat ist in der Regel ein Benzolring, der gegebenenfalls substituiert sein kann, wie etwa mit den oben genannten wasserlöslichen Gruppen.

Die Carbonylkomponente kann des Weiteren durch andere chemische Reaktionen eingeführt werden, so z.B. durch eine elektrophile Substitution an der HO- oder NH₂-Gruppe des Wirkstoffs mit einer geeigneten Carbonylkomponente.

Die so derivatisierten Wirkstoffe, die nun eine Carbonylkomponente besitzen, werden analog zu den oben beschriebenen Verfahren mit den kovalent proteinbindenden Spacermolekülen, die eine Amino-, Hydrazid- oder Hydrazingruppe besitzen, zu den entsprechenden Carboxylhydrazon-, Sulfonylhydrazon-, Hydrazon- bzw. Iminderivaten umgesetzt. Die säurelabile Spaltung dieser Bindungen führt demnach zu einer Freisetzung des derivatisierten Wirkstoffs, der eine Carbonylkomponente besitzt.

Die Spacer, die aus dem kovalent proteinbindenden Molekül PM und dem Spacermolekül SM bestehen, können z.B. gemäß Verfahren, die u.a. in DE 196 36 889 A1, U. Beyer et al. Chemical Monthly, 128, 91, 1997, R.S. Greenfield et al, Cancer Res., 50, 6600, 1990, T. Kaneko et al., Bioconjugate Chem., 2, 133, 1991, Bioconjugate Techniques, G.T. Hermanson, Academic Press, 1996 oder in US-Patent 4,251,445 beschrieben sind, hergestellt werden.

Therapeutisch wirksame Substanzen zur erfindungsgemäßen Verwendung, die ein Zytokin enthalten, können dadurch hergestellt werden, daß das Zytokin mit einem eine kovalent proteinbindende Gruppe enthaltenen Spacermolekül, das eine Carbonsäure oder eine aktivierte Carbonsäure aufweist, umgesetzt wird:

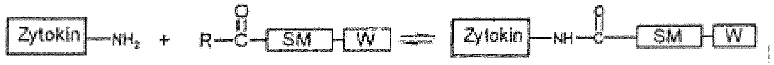

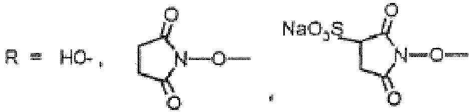

Weist das Spacermolekül eine N-Hydroxysuccinimidester-Gruppe (N-Hydroxysuccinimid oder N-Hydroxysuccinimid-3-sulfonsäure Natriumsalz) auf, wird es direkt mit dem Zytokin umgesetzt. Die Umsetzung des Zytokins mit einem eine kovalent proteinbindenden Gruppe enthaltenen Spacermolekül, das eine Carbonsäure aufweist, erfolgt in der Gegenwart eines Kondensationsmittels, wie zum Beispiel. N,N'-Dicyclohexylcarbodiimid (DCC) oder N-Cyclohexyl-N'-(2-morpholinoethyl)-carbodiimid Metho-p-toluolsulfonat (CMC), und gegebenenfalls unter Zusatz von N-Hydroxysuccinimid oder N-Hydroxysuccinimid-3-sulfonsäure Natriumsalz, zu den entsprechenden kovalent proteinbindenden Zytokinderivaten. Die Aufreinigung der so derivatisierten Zytokine erfolgt zweckmäßig mit Hilfe der Ausschlußchromatographie. Die oben beschriebenen Umsetzungen sind dem Fachmann geläufig (Bioconjugate Techniques, G.T. Hermanson, Academic Press, 1996).

Im Anschluß an die Synthese der therapeutisch wirksamen Substanz wird eine injizierbare Arzneimittelzubereitung, enthaltend die therapeutisch wirksame Substanz, in einer geeigneten Trägerflüssigkeit hergestellt. Die therapeutisch wirksame Substanz liegt bevorzugt als Lyophilisat vor, wobei vor oder nach dem Lyophilisieren übliche Träger und/oder pharmazeutische Hilfsstoffe, wie etwa Polysorbate, Glucose, Lactose, Mannose, Citronensäure, Tromethamol, Triethanolamin oder Aminoessigsäure beigefügt sein können. Die injizierbare Arzneimittelzubereitung muß so hergestellt werden, daß das kovalent proteinbindende Molekül durch das Lösen in der injizierbaren Trägerflüssigkeit nicht deaktiviert, abgespalten oder hydrolysiert wird. Weiterhin muß gewährleistet werden, daß die säurelabile Bindung in der therapeutisch wirksamen Substanz, die eine Ester-, Acetal-, Ketal-, Imin-, Hydrazon, Carboxylhydrazon- oder Sulfonylhydrazonbindung ist, nicht hydrolysiert wird. Die im Rahmen der vorliegenden Erfindung verwendeten kovalent proteinbindenden Moleküle sind basenempfindlich, so daß der pH-Wert der Trägerflüssigkeit einen pH-Wert von 8,0 nicht überschreiten soll. Bevorzugt liegt der pH-Wert im Bereich von pH 4,0-7,0, mehr bevorzugt zwischen pH 6,0 und pH 7,0. Außerdem muß die Trägerflüssigkeit natürlich physiologisch verträglich sein.

Bevorzugte Trägerflüssigkeiten sind annähernd isotonische Salzpuffer, z.B. Phosphat-, Acetat- oder Citratpuffer, wie etwa, 0.004 M Natriumphosphat, 0.15 M NaCl - pH 6,0-7,0 oder 0.01 M Natriumacetat, 0.14 M NaCl - pH 5,0-6,5). Die verwendete Trägerflüssigkeit kann auch eine isotonische Natriumchloridlösung sein. Die Salzpuffer können übliche Träger und/oder Hilfsstoffe, wie etwa Polysorbate, Glucose, Lactose, Mannose, Citronensäure, Tromethamol, Triethanolamin oder Aminoessigsäure enthalten.

Die Löslichkeit der therapeutisch wirksamen Substanz in der injizierbaren Trägerflüssigkeit kann durch pharmazeutische
Lösungsmittel, wie etwa Ethanol, Isopropanol, 1,2-Propylenglykol, Glycerol, Macrogole, Polyethylenglykole bzw. Polyethylenoxide oder durch Lösungsvermittler, z.B. Tween, Cremophor oder Polyvinylpyrrolidon, verbessert werden. Zu diesem Zweck wird die therapeutisch wirksame Substanz entweder in dem pharmazeutischen Lösungsmittel bzw. Lösungsvermittler gelöst und anschließend mit einem Salzpuffer verdünnt oder eine Trägerflüssigkeit, enthaltend den Salzpuffer und mindestens ein pharmazeutisches Lösungsmittel bzw. Lösungsvermittler, wird zum Lösen der therapeutisch wirksamen Substanz direkt verwendet. Die Konzentration der pharmazeutischen Lösungsmittel bzw. Lösungsvermittler überschreiten dabei nicht die Mengen, die vom Arzneimittelgesetz (AMG) vorgeschrieben sind.

Vorzugsweise sollte die Trägerflüssigkeit so ausgewählt werden, daß der Lösevorgang der therapeutisch wirksamen Substanz in der Trägerflüssigkeit nach wenigen Minuten abgeschlossen ist, so daß eine injizierbare Arzneimittelzubereitung am Krankenbett zur Verfügung gestellt wird.

Es kann auch zusätzlich ein Trägermolekül, wie etwa eines der eingangs genannten, mit der wirksamen Substanz in Kontakt gebracht werden, wobei die wirksame Substanz in der Lage ist, an dieses Trägermolekül zu binden. Möglich ist somit der Schritt des Zusammenbringens des Trägermoleküls und der kovalent proteinbindenden therapeutisch wirksamen Substanz ex vivo und anschließender parenterale Applikation. Auf diesem Weg kann - falls erwünscht bzw. notwendig - die Selektivität der therapeutisch wirksamen Substanz für ein Trägermolekül, beispielsweise für Albumin, verbessert werden. Die Trägermoleküle sind bevorzugt ausgewählt aus den genannten, insbesondere Serumproteinen.

Die therapeutisch wirksame Substanz zur erfindungsgemäßen Verwendung eignet sich somit zur Behandlung von Krebskrankheiten.

Die folgenden Beispiele erläutern die Erfindung näher.

### Beispiel 1

### Herstellung von DOXO-HYD

Die im folgenden abgebildete pharmakologisch aktive Substanz (abgekürzt DOXO-HYD) besteht aus dem Zytostatikum Doxorubicin, aus einer Maleinimidgruppe als kovalent proteinbindendes Molekül PM und aus einem Phenylacetylhydrazonspacer als Spacermolekül SM. Die Bindung zwischen Doxorubicin und dem Spacermolekül SM ist eine säurelabile Carboxylhydrazonbindung:

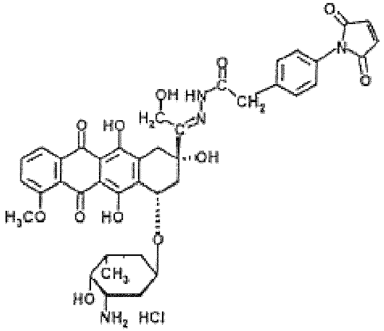

10,51 mg DOXO-HYD werden in 2,0 ml 1,2-Propylenglykol durch Schütteln gelöst und anschließend wird diese Lösung mit 8,0 ml Phosphatpuffer (0.004 M Natriumphosphat, 0.15 M NaCI - pH 6,5) verdünnt und homogenisiert (Konzentration von DOXO-HYD in der Trägerflüssigkeit ≈ 1300 µM). Die so hergestellte injizierbare Arzneimittelzubereitung von DOXO-HYD wurde Versuchstieren unmittelbar intravenös verabreicht (siehe unten).

### Beispiel 2

### Bindung von DOXO-HYD an Humanplasma

Nachdem DOXO-HYD in die Blutbahn gelangt, findet eine Bindung an Serumproteine statt, vorzugsweise an Serumalbumin, so daß DOXO-HYD u.a. als säurelabiles Albumin-Doxorubicin-Konjugat vorliegt.

Inkubationsstudien von Humanblutplasma mit DOXO-HYD bei 37 °C zeigen, daß nach einer 5-minütigen Inkubation der Großteil von DOX-HYD kovalent an Albumin gebunden ist. **(A)** - im Gegensatz zu freiem Doxorubicin **(B).** Dieser Sachverhalt ist in den untenstehenden Chromatogrammen dargestellt. **(A** und

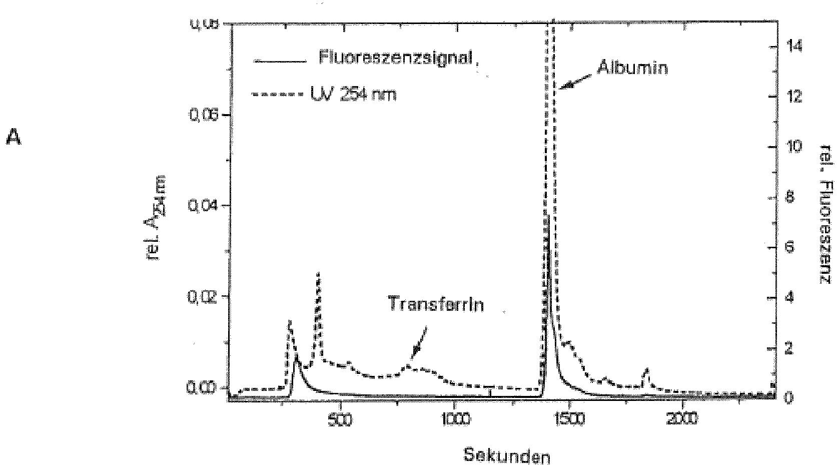

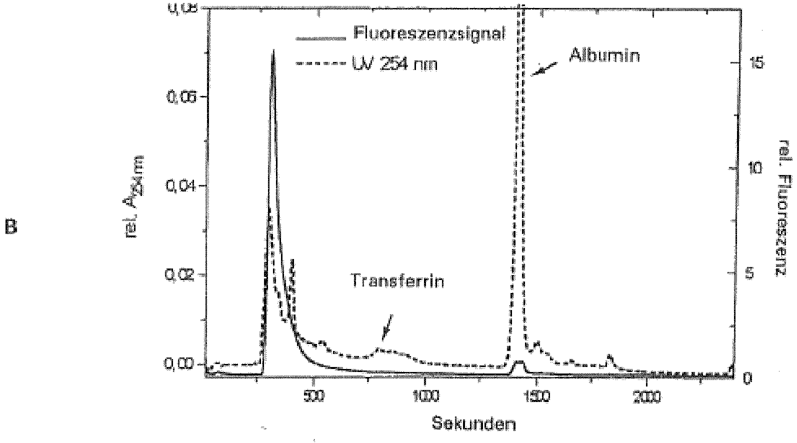

Hierbei wurde nach erfolgter Inkubation die Plasmaprobe über eine POROS®-20-Anionenaustauschersäule getrennt (Detektion der Proteine bei 254 nm, Detektion des Anthrazyklins durch Fluoreszenz).

### Beispiel 3

### Die Wirksamkeit von DOXO-HYD in vivo

Die unten aufgeführten biologischen Daten verdeutlichen die in vivo Wirksamkeit von DOXO-HYD im Vergleich zu freiem Doxorubicin: Im sogenannten RENCA (renal cell carcinoma)-Modell wurde Doxorubicin und DOXO-HYD hinsichtlich der antitumoralen Wirksamkeit bei annähernd äquitoxischer Dosis miteinander verglichen (intravenöse Therapie 10 Tage nach Injektion von etwa 1 Million Nierenkarzinomzellen in der linken Niere).
**Tiere:** Balb/c-Mäuse, weiblich; **Tumor:** RENCA, renal cell carcinoma (Nierenzellkarzinom)
**Therapie:** Tag(d) 10, 13, 17 und 20 intravenöse (i.v.), Ende des Versuchs d24

| Anzahl der Mäuse | Substanz | Dosis | Durchschnittliche Körpergewichtsabnahme (%) d 1-24 |
|---|---|---|---|
| | | | |
| 10 | Kontrolle | | |
| 10 | Doxorubicin | 4 x 10 mmol/kg | -15 |
| 10 | DOXO-HYD | 4 x 20 mmol/kg | -15 |

Die Ergebnisse dieses Versuches sind untenstehend abgebildet. DOXO-HYD zeigt eine sehr gute antitumorale Wirksamkeit und erzielt eine deutliche Reduktion des Nierentumorvolumens und der Anzahl von Lungenmetastasen im Vergleich zur Kontrollgruppe und der Doxorubicin behandelten Gruppe.

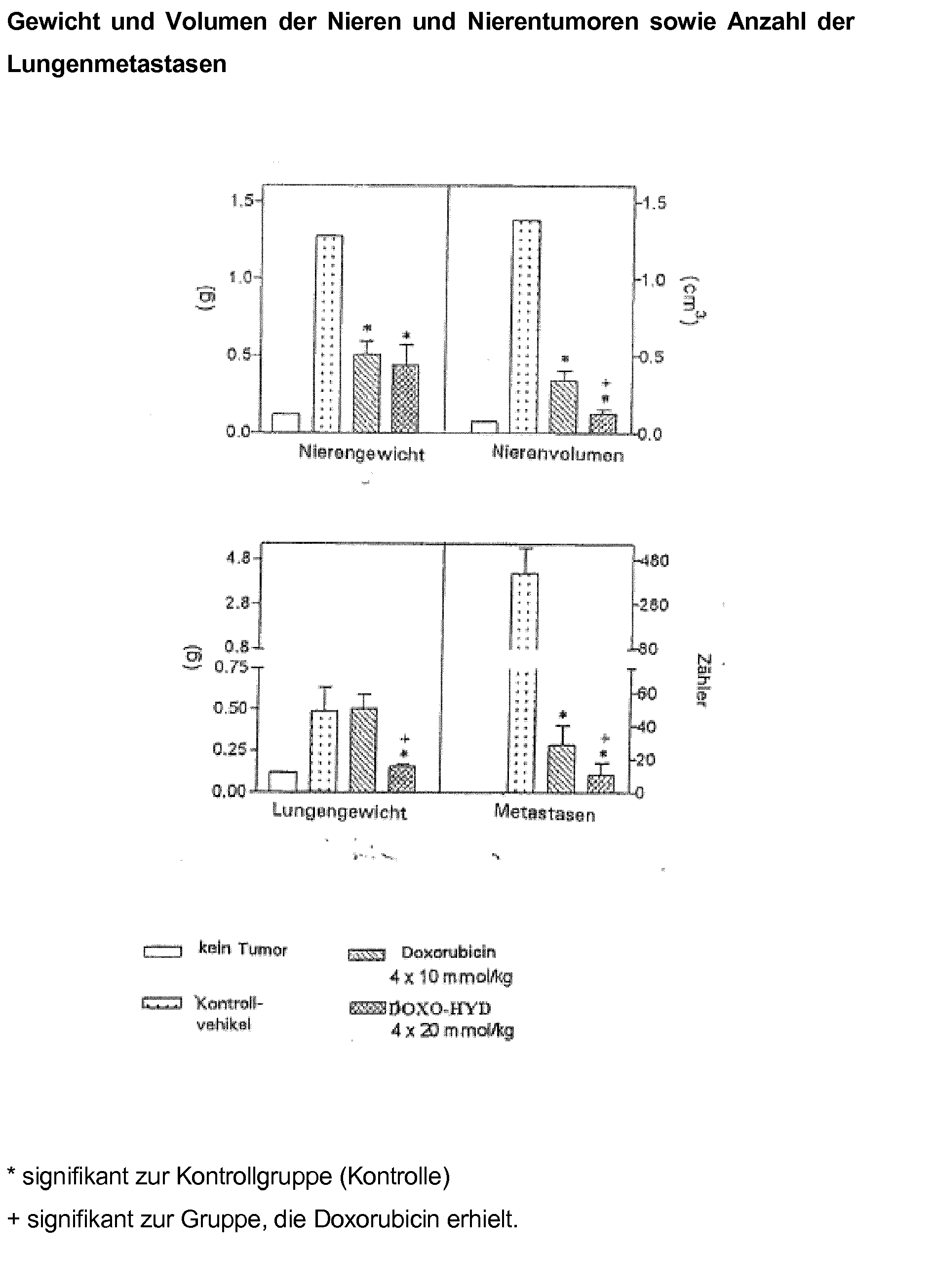

### Beispiel 4

### Bindung von DOXO-EMHC an Albumin im Humanplasma

1,6 mg des 6-Maleinimidocapronsäurehydrazon-Derivats von Doxorubicin (abgekürzt DOXO-EMHC) mit untenstehender Strukturformel 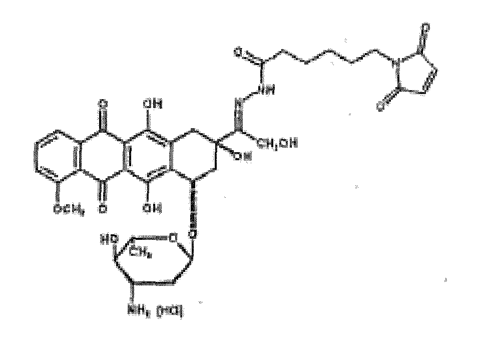 werden in 1,0 ml Phosphatpuffer (0,15 M NaCl, 0,004 M Natriumphosphat, pH 6,5) bei Raumtemperatur gelöst (2000 µM Lösung). Werden 250 µl dieser Lösung mit 1,0 ml Humanplasma 30 Sekunden lang bei 37 °C inkubiert, und die Probe anschließend über einen schwachen Anionenaustauscher (von POROS®) getrennt, zeigt sich, daß der überwiegende Teil von DOXO-EMHC an Albumin gebunden ist (s. untenstehendes Chromatogramm):

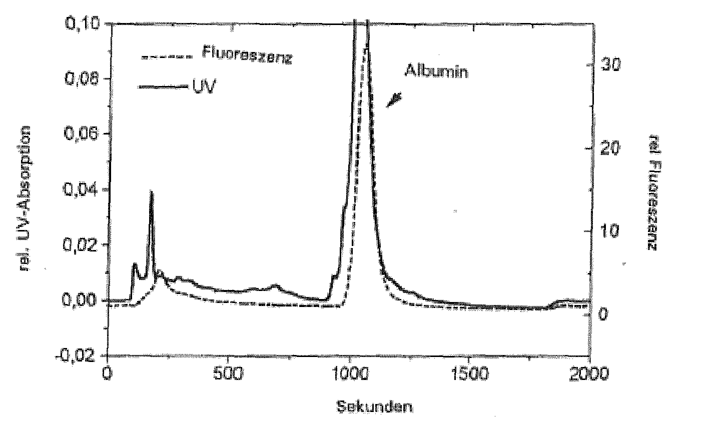

### Beispiel 5

### Bindung eines durch MMP9 spaltbares Doxorubicin-Maleinimid-Peptid-Derivats (2) an Albumin nach einminütiger Inkubation mit Humanplasma

Das Doxorubicin-Maleinimid-Peptid-Derivat (**2**) wurde gemäß folgender Reaktionsgleichung hergestellt:

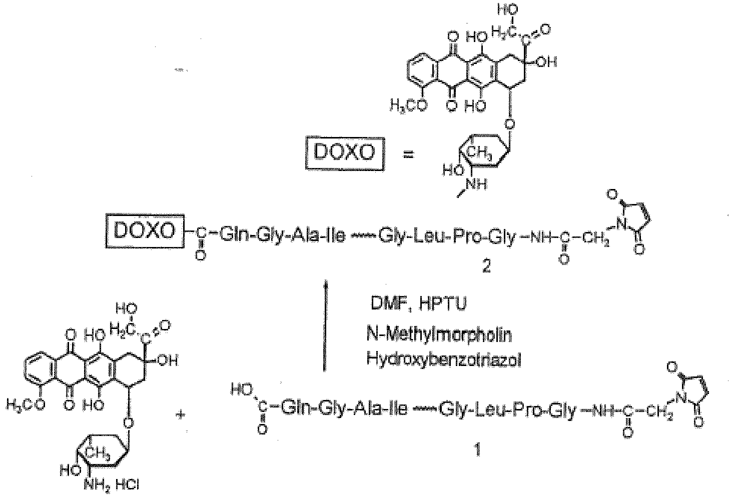

Dabei wird das mit Maleinimidoglycin derivatisierte Octapeptid Gln-Gly-Ala-Ile-Gly-Leu-Pro-Gly **1** (Mr 848, hergestellt durch Festphasensynthese durch Bachem AG, Schweiz) mit Doxorubicin laut folgender Vorschrift umgesetzt:
Zu einer leicht trüben Lösung von 17,1 mg Doxorubicin in 3 ml DMF werden 25 mg **1** (als Trifluoracetatsalz) gelöst in 500 µl DMF, 33,5 mg O-Benzotriazol-N,N,N',N'-tetramethyluroniumhexafluorphosphat (HPTU) gelöst in 200 µl DMF, 11,9 mg Hydroxybenzotriazolhydrat gelöst in 100 µl DMF und 16,2 µl N-Methylmorpholin zugegeben, und der Ansatz anschließend 18 h lang bei RT im Dunklen gerührt. DMF wurde am Hochvakuum entfernt und der Feststoff in 20 ml Methanol aufgenommen, filtriert und im Vakuum auf 1 ml eingeengt. Nach Aufreinigung über Kieselgel (Essigester/Methanol 2/1) wurden 5 mg **2** erhalten.

### Inkubationsstudie mit Humanplasma

1,4 mg **2** (Mr 1374) werden in 1,0 ml Phosphatpuffer (0,15 M NaCl, 0,004 M Natriumphosphat, pH 6,5) bei Raumtemperatur gelöst (1000 µm Lösung). Werden 300 µl dieser Lösung mit 1,0 ml Humanplasma 60 Sekunden lang bei 37 °C inkubiert, und die Probe anschließend über einen schwachen Anionenaustauscher (von POROS®) getrennt, zeigt sich, daß der überwiegende Teil von **2** an Albumin gebunden ist (s. untenstehendes Chromatogramm):

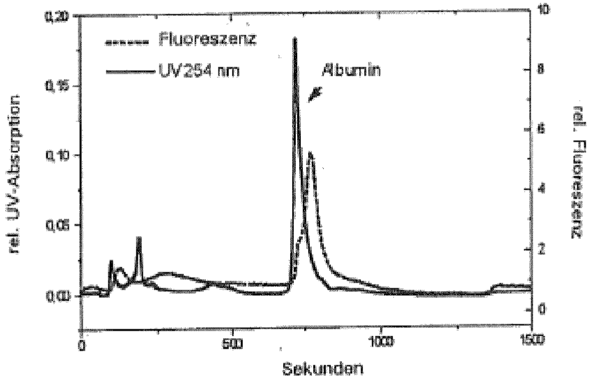

Die Peptidsequenz Gln-Gly-Ala-Ile-Gly-Leu-Pro-Gly wird von der Matrixmetalloprotease MMP9 erkannt und zwischen Isoleucin und Glycin gespalten. Dies wurde durch folgenden Versuch gezeigt: 200 µl einer 100 µM Lösung des Albuminkonjugats von **2** mit folgender Struktur (abgekürzt HSA-**2**): 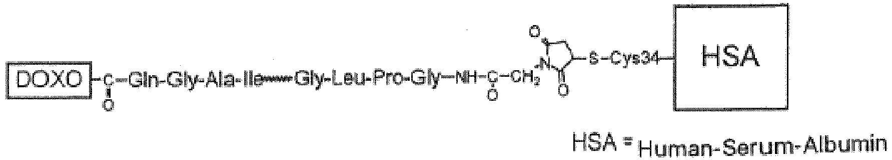 das durch das in der deutschen Patentanmeldung A19926475.9 vom 10. Juni 1999 beschriebene Verfahren hergestellt wurde, wurde mit Trypsin/Aprotinin aktivierter MMP9 (2 mU von Calbiochem, Deutschland) 30 Minuten lang bei 37 °C inkubiert. Die Freisetzung von DOXO-Gln-Gly-Ala-Ile nach dieser Zeit ist in den untenstehenden Chromatogrammen abgebildet. Gezeigt ist das Chromatogramm von HSA-**2** bei t = 0 (Trennung durch HPLC Ausschlußchromatographie mit einer Biosil 250 SEC Säule von der Firma Biorad, Detektion bei λ = 495 nm) und nach einer Inkubationszeit mit aktivierter MMP9 von 30 Minuten.

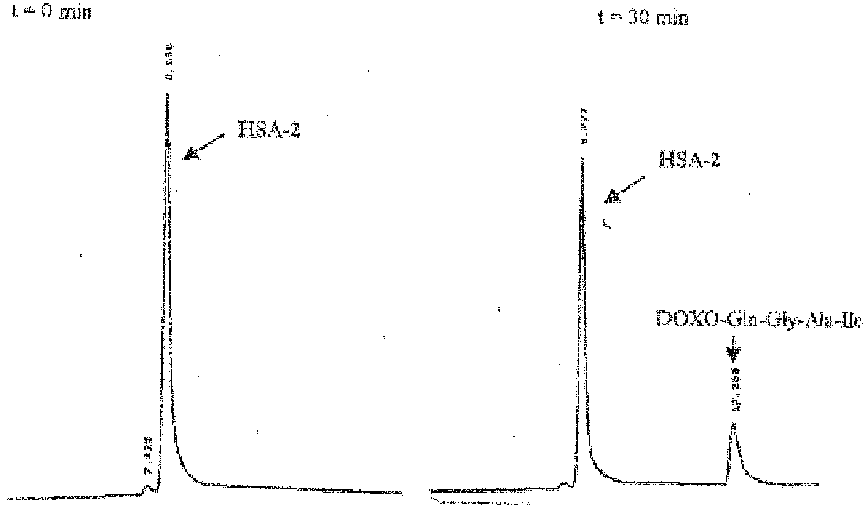

### Beispiel 6

### Bindung von Fluoresceinmaleinimid an Albumin im Humanplasma

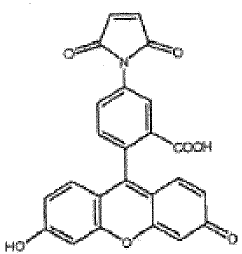

Nach einer 5-minütigen Inkubation von 250 µl einer 100 µM Fluoresceinmaleinimid-Lösung (Phosphatpuffer - 0,15 M NaCl, 0,004 M Natriumphosphat, pH 5,0) mit 1,0 ml Humanplasma und anschließenden Trennung der Probe mittels der Ausschlußchromatographie (Superdex® 200, Pharamcia), zeigt sich, daß der überwiegende Teil des Fluoresceinmaleinimids an Albumin gebunden ist (s. untenstehendes Chromatogramm):

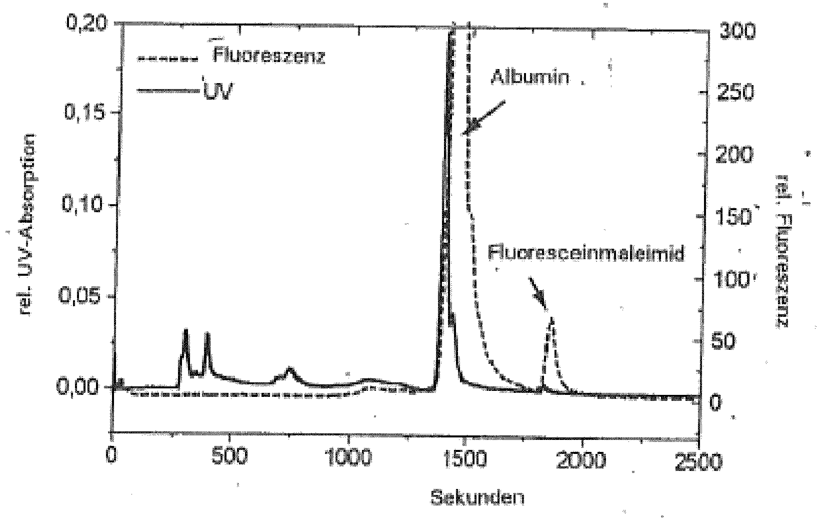

## Patentansprüche

1. Substanz, zur Verwendung als injizierbares Medikament zur Behandlung von Krebs, **dadurch gekennzeichnet, dass** die Substanz die Formel: 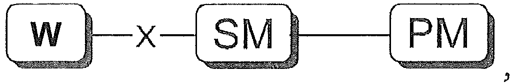 aufweist, worin
W ein Wirkstoff ist, wobei der Wirkstoff ein Zytostatikum ist,
SM ein Spacermolekül ist,
PM ein kovalent proteinbindendes Molekül ist,
X eine chemische Gruppe zwischen dem Wirkstoff und dem Spacer ist, ausgewählt aus: 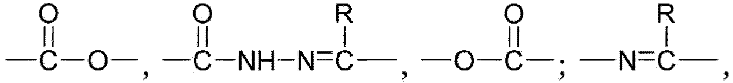 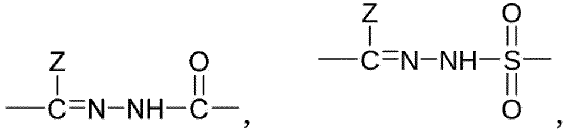 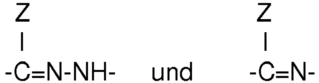 worin
R = H, Alkyl, Phenyl oder substituiertes Phenyl, und
Z = chemische Gruppe des Wirkstoffs ist.

2. Substanz zur Verwendung nach Anspruch 1, **dadurch gekennzeichnet, dass** die Substanz die Formel: 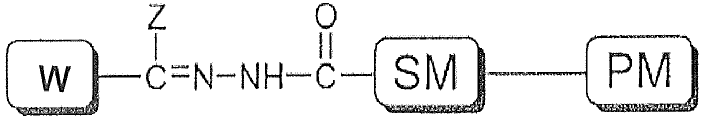 aufweist, worin W, Z, SM und PM wie in Anspruch 1 definiert sind.

3. Substanz zur Verwendung nach einem der Ansprüche 1-2, **dadurch gekennzeichnet, dass** der Wirkstoff ein Anthracyclin ist.

4. Substanz zur Verwendung nach Anspruch 3, **dadurch gekennzeichnet, dass** das Anthracyclin Doxorubicin, Daunorubicin, Epirubicin, Idarubicin, Mitoxantron oder Ametantron ist.

5. Substanz zur Verwendung nach einem der Ansprüche 1 bis 4, **dadurch gekennzeichnet, dass** das Anthracyclin Doxorubicin ist.

6. Substanz zur Verwendung nach einem der Ansprüche 1 bis 5, **dadurch gekennzeichnet, dass** der proteinbindende Rest eine Gruppe ist, ausgewählt aus einer Maleinimidgruppe, einer Halogenacetamidgruppe, einer Halogenacetatgruppe, einer Pyridyldithio-Gruppe, einer N-Hydroxysuccinimidestergruppe, einer Isothiocyanat-Gruppe, einer Disulfidgruppe, einer Vinylcarbonylgruppe, einer Aziridingruppe und einer Acetylengruppe, welche Gruppe gegebenenfalls substituiert sein kann.

7. Substanz zur Verwendung nach Anspruch 6, **dadurch gekennzeichnet, dass** der proteinbindende Molekülrest eine gegebenenfalls substituierte Maleinimidgruppe ist.

8. Substanz zur Verwendung nach einem der Ansprüche 1 bis 7, **dadurch gekennzeichnet, dass** der Spacer ein organischer Molekülrest ist, welcher eine aliphatische Kohlenstoffkette und/oder einen aliphatischen Kohlenstoffring mit 1-12 Kohlenstoffatomen, die teilweise durch Sauerstoffatome ersetzt sein können, und/oder mindestens einen Aromaten enthält, welche ggf. substituiert sein können.

9. Substanz zur Verwendung nach Anspruch 8, **dadurch gekennzeichnet, dass** der Spacer wenigstens eine gegebenenfalls substituierte aliphatische Kohlenstoffkette, umfassend 1 bis 12 Kohlenstoffatome, umfasst.

10. Substanz zur Verwendung nach einem der Ansprüche 1 bis 9, **dadurch gekennzeichnet, dass** die Substanz 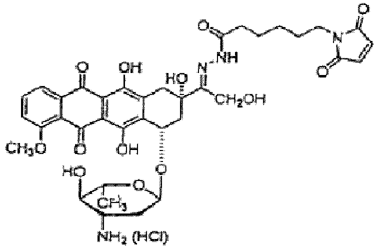 ist.

11. Zusammensetzung zur Verwendung als injizierbares Medikament zur Behandlung von Krebs, umfassend eine Substanz nach einem der Ansprüche 1-10 und eine Trägerflüssigkeit, wobei die Substanz nach ihrer Verwendung kovalent an eine Körperflüssigkeit bindet.

## Claims

1. Substance for use as an injectable medicament for the treatment of cancer, **characterised in that** the substance has the formula: 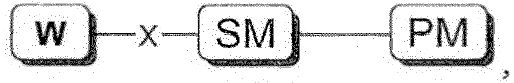 in which
W is an active ingredient, where the active ingredient is a cytostatic agent,
SM is a spacer molecule,
PM is a covalently protein-binding molecule,
X is a chemical group between the active ingredient and the spacer, selected from: 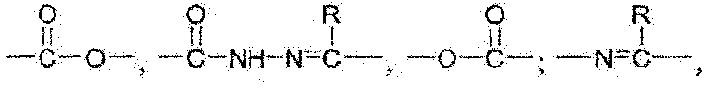 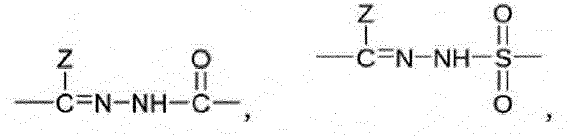 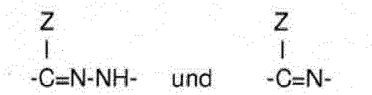 in which
R = H, alkyl, phenyl or substituted phenyl, and
Z = chemical group of the active ingredient.

2. Substance for use according to claim 1, **characterised in that** the substance has the formula: 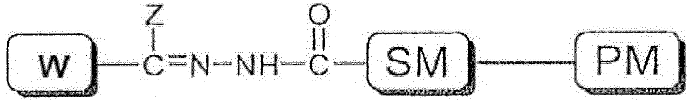 in which W, Z, SM and PM are as defined in claim 1.

3. Substance for use according to either claim 1 or claim 2, **characterised in that** the active ingredient is an anthracycline.

4. Substance for use according to claim 3, **characterised in that** the anthracycline is doxorubicin, daunorubicin, epirubicin, idarubicin, mitoxantrone or ametantrone.

5. Substance for use according to any of claims 1 to 4, **characterised in that** the anthracycline is doxorubicin.

6. Substance for use according to any of claims 1 to 5, **characterised in that** the protein-binding group is a group selected from a maleimide group, a haloacetamide group, a haloacetate group, a pyridyldithio group, an N-hydroxysuccinimide ester group, an isothiocyanate group, a disulfide group, a vinylcarbonyl group, an aziridine group and an acetylene group, which group may be optionally substituted.

7. Substance for use according to claim 6, **characterised in that** the protein-binding molecular residue is an optionally substituted maleimide group.

8. Substance for use according to any of claims 1 to 7, **characterised in that** the spacer is an organic molecular residue which contains an aliphatic carbon chain and/or an aliphatic carbon ring having 1-12 carbon atoms, which may be partially replaced by oxygen atoms, and/or at least one aromatic which may be optionally substituted.

9. Substance for use according to claim 8, **characterised in that** the spacer comprises at least one optionally substituted aliphatic carbon chain comprising 1 to 12 carbon atoms.

10. Substance for use according to any of claims 1 to 9, **characterised in that** the substance is

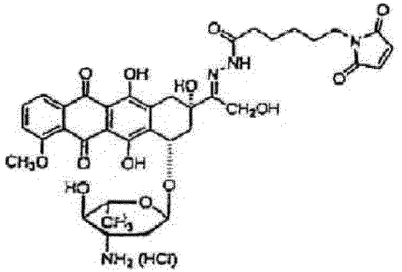

11. Composition for use as an injectable medicament for the treatment of cancer, comprising a substance according to any of claims 1-10 and a carrier liquid, wherein the substance covalently binds to a body fluid after use.

## Revendications

1. Substance pour l'utilisation comme médicament injectable pour le traitement du cancer, **caractérisée en ce que** la substance présente la formule : 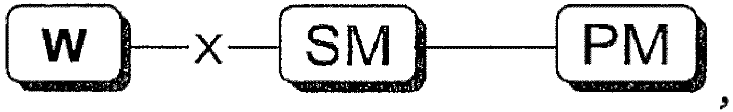 dans laquelle
W est une substance active, la substance active étant un cytostatique,
SM est une molécule d'espaceur,
PM est une molécule se liant de façon covalente à une protéine,
X est un groupe chimique entre la substance active et l'espaceur, choisi parmi : 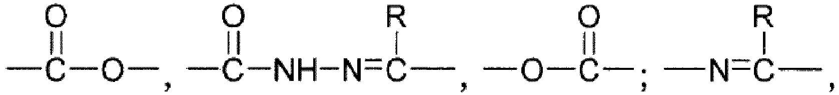 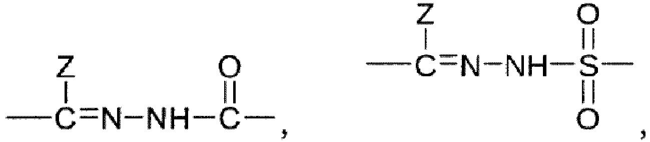 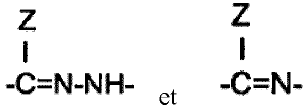 dans laquelle
R = H, un groupe alkyle, phényle ou phényle substitué, et
Z = un groupe chimique de la substance active.

2. Substance pour l'utilisation selon la revendication 1, **caractérisée en ce que** la substance présente la formule : 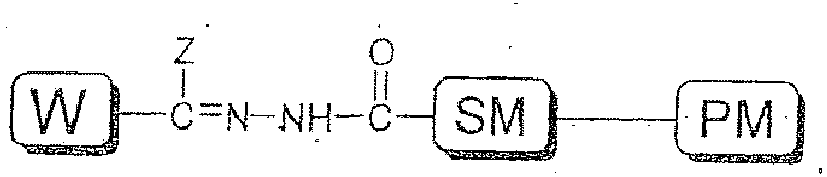 dans laquelle W, Z, SM et PM sont tels que définis dans la revendication 1.

3. Substance pour l'utilisation selon l'une des revendications 1 à 2, **caractérisée en ce que** la substance active est une anthracycline.

4. Substance pour l'utilisation selon la revendication 3, **caractérisée en ce que** l'anthracycline est la doxorubicine, la daunorubicine, l'épirubicine, l'idarubicine, la mitoxantrone ou l'amétantrone.

5. Substance pour l'utilisation selon l'une des revendications 1 à 4, **caractérisée en ce que** l'anthracycline est la doxorubicine.

6. Substance pour l'utilisation selon l'une des revendications 1 à 5, **caractérisée en ce que** le résidu se liant à une protéine est un groupe choisi parmi un groupe maléinimide, un groupe halogéno-acétamide, un groupe halogéno-acétate, un groupe pyridyldithio, un groupe N-hydroxysuccinimide ester, un groupe isothiocyanate, un groupe disulfure, un groupe vinylcarbonyle, un groupe aziridine et un groupe acétylène, ledit groupe pouvant éventuellement être substitué.

7. Substance pour l'utilisation selon la revendication 6, **caractérisée en ce que** le résidu moléculaire se liant à une protéine est un groupe maléinimide éventuellement substitué.

8. Substance pour l'utilisation selon l'une des revendications 1 à 7, **caractérisée en ce que** l'espaceur est un résidu moléculaire organique, qui contient une chaîne carbonée aliphatique et/ou un cycle carboné aliphatique avec 1 à 12 atomes de carbone qui peuvent être partiellement remplacés par des atomes d'oxygène, et/ou qui contient au moins un composé aromatique qui peuvent éventuellement être substitués.

9. Substance pour l'utilisation selon la revendication 8, **caractérisée en ce que** l'espaceur comprend au moins une chaîne carbonée aliphatique éventuellement substituée, comprenant 1 à 12 atomes de carbone.

10. Substance pour l'utilisation selon l'une des revendications 1 à 9, **caractérisée en ce que** la substance est

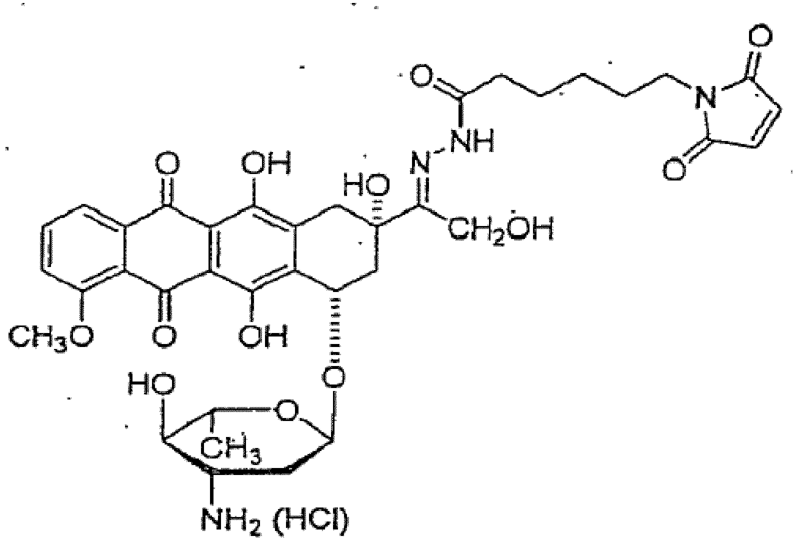

11. Composition pour l'utilisation en tant que médicament injectable pour le traitement du cancer, comprenant une substance selon l'une des revendications 1 à 10 et un liquide de support, la substance se liant de façon covalente à un fluide corporel après son utilisation.
